Europäisches Patentamt

European Patent Office

Office européen des brevets

(11) Publication number: **0 369 775**
**A2**

(12) **EUROPEAN PATENT APPLICATION**

(21) Application number: **89311834.9**

(22) Date of filing: **15.11.89**

(51) Int. Cl.5: **C12Q 1/68, C12N 15/10,**
**//C07H21/04**

(30) Priority: **18.11.88 US 273457**

(43) Date of publication of application:
**23.05.90 Bulletin 90/21**

(84) Designated Contracting States:
**AT BE CH DE ES FR GB GR IT LI LU NL SE**

(71) Applicant: **LIFECODES CORPORATION**
**Saw Mill River Road**
**Valhalla New York 10595(US)**

(72) Inventor: **Loewy, Zvi G.**
**4-50 Lyncrest Avenue**
**Fairlawn New Jersey 07410(US)**
Inventor: **Baum, Howard J.**
**18 Stubbe Drive**
**Stony Point New York 10980(US)**

(74) Representative: **Perry, Robert Edward et al**
**GILL JENNINGS & EVERY 53-64 Chancery**
**Lane**
**London WC2A 1HN(GB)**

(54) **Compositions and process for amplifying and detecting nucleic acid sequences.**

(57) Compositions and methods useful for detecting, amplifying and/or characterizing polynucleotides of interest are disclosed herein. Specifically, this disclosure teaches how to make and use a mobile promoter structure comprising a transcription promoter and a probe sequence. The probe sequence enables the mobile promoter structure to select via hybridization a specific polynucleotide of interest. The promoter enables the transcription of the target polynucleotide. The method of mobile promoter structure-directed transcription can be applied to detect, and/or characterize pathogens, disease states, and genetic traits. While a single mobile promoter structure-directed transcription enables linear amplification, the process can be used in combination with other processes to bring about exponential amplification.

EP 0 369 775 A2

## COMPOSITIONS AND PROCESS FOR AMPLIFYING AND DETECTING NUCLEIC ACID SEQUENCES

### Background of the Invention

For many applications, it is desirable to amplify a nucleic acid sequence of interest. A first example is in the area of diagnostics. Amplification of a nucleic acid sequence specific for an infectious agent would permit greater sensitivity of detection. Indeed, the amplification process itself may serve as a detection means. A second example is where a large quantity of a nucleic acid sequence is needed for studies of its structure and function. Numerous other examples can be found in the literature.

Several methods have been described in the literature for the synthesis of nucleic acids de novo or from an existing sequence. These methods are capable of producing large amounts of a given nucleic acid of completely specified sequence. But if a nucleic acid sequence of interest is not completely specified by demonstration or otherwise, these methods do not permit selective amplification of a nucleic acid sequence of interest among other nucleic acid sequences not of interest.

One known method for synthesizing nucleic acids de novo involves the organic synthesis of a nucleic acid from nucleoside derivatives. The synthesis can be performed in solution or on a solid support. One type of organic synthesis is the phosphotriester method. For a description of this method, see Narang et al., Meth. Enzymol. 68: 90 (1979) and U.S. Patent No. 4,356,270. A second type of organic synthesis is the phosphodiester method. For a description of this method, see Brown et al., Meth. Enzymol. 68: 109 (1979). Both the phosphotriester and the phosphodiester methods can be used to prepare oligonucleotides which are then joined together to form genes of significant size. However, these processes are laborious and time-consuming, require expensive equipment and reagents, and have a low over-all efficiency. Thus, these methods are not practical for synthesizing large amounts of any desired nucleic acid.

Methods also exist for producing nucleic acids in large amounts from small amounts of an existing nucleic acid of interest. These methods involve recombinant nucleic acid technology. Thus, a second method is carried out by inserting a nucleic acid of interest into an appropriate cloning vector. The recombinant containing the nucleic acid of interest is then introduced into an appropriate host. Copies of the nucleic acid of interest are produced in the host via replication and/or transcription of the recombinant. For a description of these methods, see, for example, Maniatis et al., Molecular Cloning: A Laboratory Manual, Cold Spring Harbor Laboratory, pp. 390-401 (1982), U.S. Patent Nos. 4,416,988 and 4,403,036. A drawback of this method is that the nucleic acid of interest must be separated from nucleic acids not of interest either before or after cloning.

A third method for synthesizing nucleic acids, a combination of organic synthesis and cloning, is described in U.S. Patent No. 4,293,652. In this process, the appropriate oligonucleotides are organically synthesized and sequentially inserted into a vector. A recombinant formed in an earlier insertion is amplified prior to the next insertion.

A fourth method for producing nucleic acid sequences is based on transcription. Multiple copies of a nucleic acid sequence can be obtained by transcription if it is properly positioned downstream from an appropriate promoter sequence. See, for example, Melton et al., Nucl. Acids Res. 12: 4035 (1984). More recently, Milligan et al., Nucleic Acids Res. 15: 8783 (1987) taught transcription off a template which is largely single-stranded. Here again, the prior art methods teach amplified production of a completely specified nucleic acid sequence, but does not teach selective synthesis of a nucleic acid sequence of interest among other nucleic acid sequences not of interest. Moreover, Milligan et al. did not teach synthesis of RNA of extended length.

A fifth method for synthesizing nucleic acids is based on repetitive, enzymatic, primed synthesis of a nucleic acid sequence, also known as Polymerase Chain Reaction ("PCR") (see U.S. 4,683,195). A nucleic acid sequence of interest is duplexed with a primer which is one end of the complement to the nucleic acid of interest. A first primer extension product is produced by enzymatic synthesis. The first primer extension product is now available as a new template for the next round of primer extension synthesis. Indeed, a second primer which is one end of the nucleic acid sequence of interest is duplexed to the new template, and primes the synthesis of a second extension product which is a copy of the nucleic acid sequence of interest. If the reaction conditions are such that the primers, the substrates and the enzymes are not limiting, the amount of nucleic acid sequence of interest and its complement will grow as an exponential function of the number of primer extension synthesis cycles.

PCR does not require synthesis of nucleic acid sequences unrelated to the nucleic acid sequence of interest or its complement. Moreover, it is not necessary to specify completely the nucleic acid sequence of interest; it is only necessary to specify its two ends. For example, the method is applicable where the 5' and 3' ends of a gene, but not the internal sequence, are known. Large quantities of the gene can be made by this method starting only with total genomic DNA. However, there are serious drawbacks to the PCR method as well. For unknown reasons, PCR does not work efficiently where the sequence internal to the primers approaches or exceeds one kilobase in length. Finally, this method necessarily causes the amplified synthesis of the complement of the nucleic acid sequence of interest, and therefore, does not permit unidirectional amplification, i.e., amplification of only one strand of a gene.

## Brief Summary of the Invention

The present invention relates to compositions and a process for amplifying and/or detecting nucleic acid sequences. Specifically, it relates to compositions and a process for producing from a specific nucleic acid sequence copies of the sequence in amounts which are large compared to the amount initially present. The initial nucleic acid may be DNA or RNA. It may be single- or double-stranded. It may be a relatively pure species or a component of a mixture of nucleic acids. The amplification process of the invention utilizes a repetitive reaction to accomplish the amplification of the nucleic acid sequence. Because the repetitive reaction is critically dependent on the presence of the specified nucleic acid sequence, the process of the present invention can also serve as a means of detecting the specified nucleic acid sequence.

More specifically, the present invention provides a "mobile promoter structure" linked to a polynucleotide probe which duplexes with a target nucleic acid of interest. The target nucleic acid of interest is amplified by transcription under the control of the mobile promoter structure. Further amplification of the target nucleic acid of interest, and other applications of this method are described infra. The present invention bears some similarity to the fourth and fifth methods described in the Background of the Invention, supra. However, it improves on the fourth method in (a) that it does not require complete specification of the nucleic acid sequence of interest, (b) that it permits the selective synthesis of the nucleic acid sequence of interest among other nucleic acid sequences not of interest, and in other aspects. It improves on the fifth method in (a) that it permits unidirectional synthesis of a gene, (b) that sequence information at only one end of the nucleic acid sequence of interest need be specified, and in other aspects.

The advantages of the subject invention polymerase/transcription system for amplification of nucleic acid sequences, as compared to the PCR system, can be summarized as follows:

    a. Longer target sequences can be transcribed in the invention system, than can be replicated in PCR.

    b. Nucleic acid sequences can be doubled every cycle in PCR. Polymerase/transcription - nucleic acid sequence can be increased by 100X.

    c. Time of each cycle is shorter for polymerase/transcription than for PCR.

    d. A thermostable enzyme is not required in the invention system.

    e. Polymerase/transcription elicits enhanced specificity over PCR.

    f. Polymerase/transcription system will work on RNA and DNA. PCR has been demonstrated only on DNA (unless RNA is reverse transcribed with an additional enzyme).

    g. Polymerase/transcription - does not require heat denaturation every cycle, which can result in nicking the DNA.

## Brief Description of the Drawings

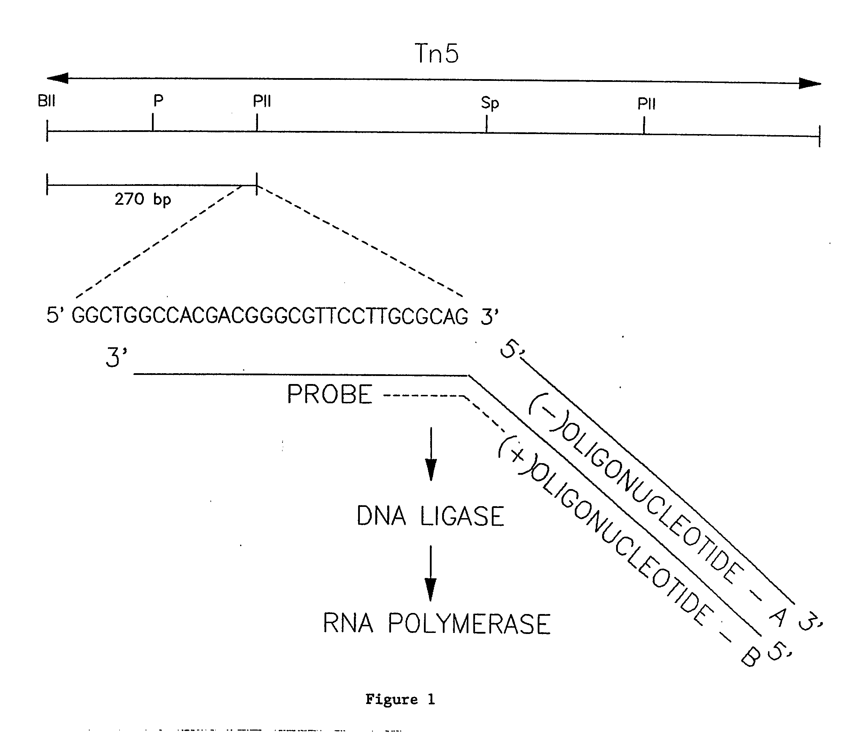

Figure 1 shows the generalized structure and the process of formation of a mobile promoter structure -Tn5 neo gene annealed complex. A partial restriction map of the neo gene is represented at the top of the figure. The target sequence corresponds to the 270 bp BglII/PvuII fragment at the 5' region of the gene. The DNA sequence of the target region that is complementary to the probe sequence of the mobile promoter structure is shown. Two complementary oligonucleotides (A and B) which constitute a promoter and a probe sequence are annealed to the target template. The mobile promoter structure is ligated to the target template and subsequently transcribed by a RNA polymerase. The top oligonucleotide corresponds to the (-) promoter strand and the bottom oligonucleotide corresponds to the (+) promoter strand.

Figure 2 demonstrates the formation of a ligated annealed complex. The ligated complex is detected by polyacrylamide gel analysis.

Figure 3 shows an electrophoretic analysis of the transcription products off the T3 mobile promoter structure-Tn5 neo gene annealed complex.

Figure 4 illustrates a) the structure of a "cloned" T3 polymerase unit; and b) a T3-mobile promoter structure transcription complex.

Figure 5 demonstrates the magnitude of amplification obtained with the mobile promoter structure as compared to primer extension synthesis.

Figure 6 illustrates the specificity of the T3 mobile promoter structure in a background of total human genomic DNA.

Figure 7 illustrates the ligated annealed complexes where the templates (target sequences) are a) the wild type HaeIII-Hinfl beta globin fragment; and b) the wild type HaeIII-Ddel beta globin fragment.

Figure 8 shows an electrophoretic analysis of the transcription products obtained off of an RNA template.

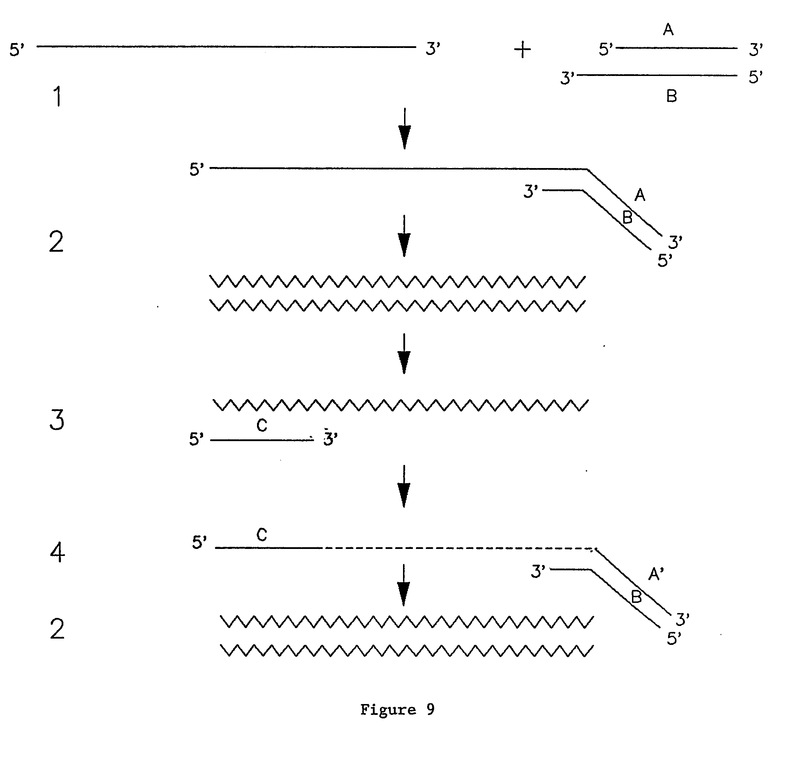

Figure 9 illustrates the general scheme of multiple alternating cycles of mobile promoter structure directed transcription, and reverse transcription by primer extension synthesis, by which a polynucleotide of interest can be amplified exponentially relative to the number of cycles of synthesis. The intense dark line corresponds to a target sequence of interest. The mobile promoter structure, which is comprised of oligonucleotides A and B, is annealed and ligated to the target sequence (Step 1). Transcription with an appropriate RNA polymerase results in multiple transcripts (wavy lines; Step 2). To copy the transcripts, a primer (oligonucleotide C) is annealed to the 3′ end of the transcript (Step 3), and is extended upon the addition of deoxynucleotides and a DNA polymerase. A second mobile promoter structure comprising oligonucleotides A′ and B is annealed and ligated to the primer extension (Step 4). This mobile promoter structure is devoid of the bridge sequence. Multiple cycles of this process result in an exponential amplification of the initial target sequence. This scheme is also applicable to the situation where a bridge sequence is not necessary for efficient transcription. In this case, oligonucleotide A′ would substitute for oligonucleotide A throughout the iterative process.

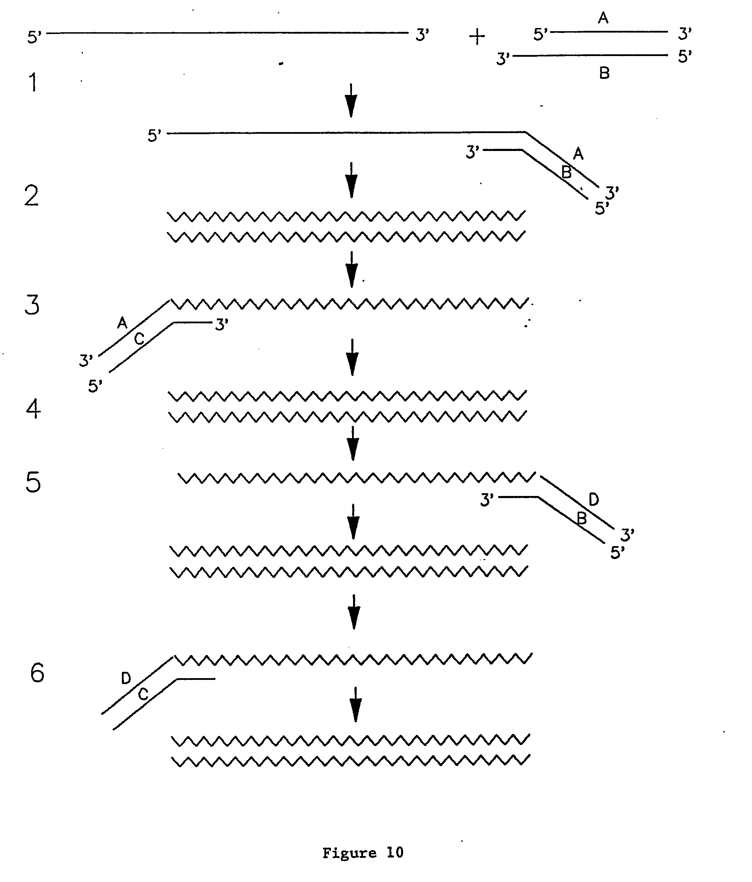

Figure 10 illustrates a general scheme of multiple rounds of mobile promoter structure directed transcription. The intense dark line corresponds to a target sequence of interest. The mobile promoter structure, which is comprised of oligonucleotides A and B, is annealed and ligated to the target sequence (Step 1). Transcription with an appropriate RNA polymerase results in multiple transcripts (wavy lines; Step 2). The transcripts are annealed and ligated to a second mobile promoter structure comprising oligonucleotides A and C (Step 3). Transcription of the RNA template results in the synthesis of complementary transcripts (Step 4). These transcripts are annealed and ligated to a third mobile promoter comprising oligonucleotides D and B (Step 5). This mobile promoter structure is devoid of the bridge sequence. Transcription of this RNA template results in the synthesis of complementary transcripts to the RNA of Step 4. These transcripts are subsequently annealed and ligated to a fourth mobile promoter structure, comprising oligonucleotides D and C, which is also devoid of a bridge sequence (Step 6). This process can be reiterated, resulting in an exponential amplification of the initial target sequence. This scheme is also applicable to the situation where a bridge sequence is not necessary for efficient transcription. In this case, oligonucleotide D would substitute for oligonucleotide A throughout the iterative process.

## Detailed Description of the Invention

Following are various terms used in this disclosure and their intended meaning:

### PROMOTER

A double-stranded polynucleotide sequence which is essential for and controls the initiation of transcription. A promoter operates asymmetrically. Consider the double-stranded polynucleotide sequence:

5′-A....BX..YZC.....D-3′
3′-a....bx..yzc.....d-5′

where "A" base pairs with "a" according to the Watson-Crick scheme, "B" with "b", etc.; and

5′-X..YZ-3′
3′-x..yz-5′

is a promoter.

4

Because the promoter operates asymmetrically, an RNA polymerase will transcribe <u>only one</u> strand in the 5′ to 3′ direction.

For the purposes of this application, the following convention is adopted:

(1) a promoter will be generically denoted by

5′-X..YZ-3′
3′-x..yz-5′ ;

(2) the transcription direction goes from X to Z, not from z to x;

(3) the strand (or sequence) 5′-A...BX..YZC...D-3′is the plus strand (or sequence);

(4) the strand (or sequence) 3′-a...bx..yzc...d-5′is the minus strand (or sequence);

(5) the sequence 5′-X..YZ-3′ is the "plus promoter" sequence; and

(6) the sequence 3′-x..yz-5′ is the "minus promoter" sequence.

The first nucleotide (i.e. the 5′-terminal nucleotide) of a transcript corresponds to the first nucleotide on the plus strand downstream from (i.e. on the 3′ side of) the promoter.

PROBE SEQUENCE

A polynucleotide of such length and composition that a duplex formed between it and a substantially complementary polynucleotide will be stable under conditions which permit transcription. In this application, a probe will be generically denoted by 5′ D....EF 3′, or 3′-fe...d-5′. The length of the polynucleotide may be within a range which overlaps the range of oligonucleotides.

TARGET POLYNUCLEOTIDE

A target polynucleotide of a probe is a polynucleotide which is substantially complementary to the probe so that a duplex formed between the two is stable under conditions which permit transcription. In this application, a target polynucleotide will be generically denoted by 3′-d...efp.....qr-5′, or 3′-lmnd....efp....qr-5′. When a probe sequence and its target polynucleotide is annealed to form a duplex, the 3′ terminal nucleotide of the target polynucleotide of a probe very preferably base pairs or "mismatches" the 5′ terminal nucleotide of the probe sequence. It is very highly preferred that there is no mismatch.

BRIDGE SEQUENCE

A oligo- or polynucleotide bridge between a probe sequence and a promoter. The presence of a bridge sequence is not essential, but facilitates the synthesis of full length transcripts. Thus, the bridge sequence can be zero or more nucleotides long.

MOBILE PROMOTER STRUCTURE

A mobile promoter structure comprises a promoter and a probe sequence. The mobile promoter structure may also contain a bridge sequence. Further, the mobile promoter structure may be attached to a target polynucleotide of the probe sequence by hybridization. The hybridized structure is transcriptionally competent, and the target polynucleotide becomes the transcription template, or a part thereof.

+1 NUCLEOTIDE, +1′ NUCLEOTIDE

The first nucleotide of a transcript corresponds to a nucleotide on the plus strand of the transcription complex, which is immediately next to the 3′ end of the plus promoter sequence. The nucleotide to which the first nucleotide of the transcript corresponds is called the +1 nucleotide. The nucleotide on the minus strand which base pairs with the +1 nucleotide is the +1′ nucleotide.

In one embodiment of the present invention, a first polynucleotide sequence comprising three polynucleotide sequences linked in a 5′ to 3′ direction in this order - (1) a plus promoter sequence, (2) a "plus" bridge sequence, and (3) a probe sequence - is duplexed with a second polynucleotide sequence comprising two polynucleotide sequences linked in a 3′ to 5′ direction in this order - (1) a minus promoter sequence, and (2) a "minus" bridge sequence, wherein the plus promoter sequence is complementary to the minus promoter sequence, and the "plus" bridge sequence is complementary to the "minus" bridge sequence, to form the following partially double-stranded polynucleotide structure:

$$\wedge\wedge 5'\text{-X..YZ}(I)_nD.....EF\text{-}3'$$
$$\sim 3'\text{-x..yz}(i)_n \quad (A)$$

where:

(1) $\wedge\wedge$ stands for the remaining portion of the first polynucleotide sequence;

(2) $\sim$ stands for the remaining portion of the second polynucleotide sequence;

(3) I is a nucleotide; i is a nucleotide which base pairs with I;

(4) n is greater than or equal to 0;

(5) $(I)_n$ is the "plus" bridge sequence, and $(i)_n$ is the "minus" bridge sequence complementary to $(I)_n$;

(6) $5'\text{-X..YZ-}3'$

$3'\text{-x..yz-}5'$ is a promoter; and

(7) $5'\text{-D....EF-}3'$ is a probe sequence. The first polynucleotide sequence has a $3'$ end which is also the $3'$ end of the probe sequence. The second polynucleotide sequence has a $5'$ end which is the $5'$ end of the "minus" bridge sequence if n > 0, or the $5'$ end of the promoter if n = 0 in formula (A). The structure of formula (A) does not support transcription except synthesis of $(I)_n$, but can serve as a "mobile promoter structure" as described in greater detail below.

With some mobile promoter structures, it is preferred that there is a specific bridge sequence. Thus, the +1 nucleotide is several bases away from the $5'$ end of the probe sequence. This provision appears to decrease abortive attempts in transcription, and facilitates the synthesis of full transcripts when the promoter is attached to a transcription template.

$\wedge\wedge$ and $\sim$ may comprise moieties other than nucleotides, e.g., reporter molecules. $\wedge\wedge$ and $\sim$ may be covalently linked so that the first and second polynucleotide sequences are two parts of a larger polynucleotide sequence. If this is the case, the duplex comprising $5'\text{-X..YZ}(I)_n\text{-}3'$ will be formed automatically under

$3'\text{-x..yz}(i)_n\text{-}5'$

conditions which permit hybridization of the complementary sequences. Otherwise, the first and second polynucleotide sequences must be brought together under said conditions to form the structure of formula (A).

In another embodiment of the invention, a mobile promoter structure is annealed to a target polynucleotide of interest to form an annealed complex which is transcriptionally competent, i.e., the complex will support transcription of the target polynucleotide of interest by an RNA polymerase compatible with the mobile promoter structure under the control of the mobile promoter structure. (Note that a RNA dependent RNA polymerase would be expected to prefer a RNA annealed complex. Likewise, a DNA dependent RNA polymerase would be expected to prefer a DNA annealed complex. However, there is no absolute requirement as to the chemical nature of the complex.)

The chemical structure of an annealed complex is shown below in formula (B):

(B)

$$\wedge\wedge 5'\text{-X..YZ}(I)_nD....EF\text{-}3'$$
$$\sim 3'\text{-x..yz}(i)_n\ PO_4\ VW\text{-}3'd....efp.........qr\text{-}5'$$

where $5'\text{-WVd....epf......qr-}5'$ is the target polynucleotide; and the other symbols have the same meaning as in formula (A). (A full length transcript of the annealed complex (B) would be $5'\text{-}(I)_nD.....EFP..........QR\text{-}3'$.)

The probe sequence on the mobile promoter structure forms a duplex with a target polynucleotide of interest. The length and composition of the probe sequence are such that the duplex is stable under conditions which will support the transcription process. For example, the duplex must not melt in the transcription reaction mixture. Otherwise, a certain degree of mismatch between the probe sequence and the target polynucleotide can be tolerated. Mismatches are tolerated probably because the RNA polymerase reads the template strand which is the target polynucleotide of interest (which is the continuation of the minus strand), and synthesizes a transcript which begins at the first nucleotide, and is complementary to the template strand. Mismatches between the probe sequence and the target polynucleotide would not disrupt this process.

In a preferred embodiment, the $5'$ terminal nucleotide of the minus promoter sequence, or the minus bridge sequence, as the case may be, is immediately next to the $3'$ terminal nucleotide of the target nucleotide on the same double helix. Thus, in a preferred embodiment, the structure (B) would look like:

$$\wedge\wedge 5'\text{-X..YZ}(I)_nD....EF\text{-}3'$$
$$\sim 3'\text{-x..yz}(i)_n PO_4\text{-}5'\ OH\text{-}3'd....efp.........qr\text{-}5' \quad B'$$

where $3'\text{-d....efp......qr-}5'$ is the target polynucleotide; and the other symbols have the same meaning as in formula (A).

In a more preferred embodiment, the annealed complex is ligated to form a ligated annealed complex. In the annealed complex, there is a nick between the 5' terminal nucleotide on the second polynucleotide sequence of the mobile promoter structure and the 3' terminal nucleotide of the target polynucleotide of interest. (See formula (B'). In the ligated annealed complex, the nick is repaired, and the template is a continuous strand from the +1' nucleotide through the junction between the mobile promoter to the 5' end of the target polynucleotide.

While both the ligated annealed complex and the unligated annealed complex will support transcription, full length transcripts are synthesized much more efficiently with the ligated annealed complex.

It should be noted that the mobile promoter structure need not be formed prior to annealing with the target polynucleotide of interest. Thus, the first polynucleotide sequence and the second polynucleotide sequence of a mobile promoter structure, and a target polynucleotide of interest may be brought into a single reaction mixture where they form the annealed complex.

Because many copies of transcripts can be made from each transcriptionally competent complex, the structure of formula (B) or (B'), or (B') in ligated form can be used to amplify the target polynucleotide of interest, or portions thereof. The bridge sequence on the complex, i.e. (I)$_n$, if present, would be co-amplified with the sequence 5'-D...EF....

The target polynucleotide of interest can be in a mixture comprising other polynucleotides not of interest. The mobile promoter structure manifests selectivity: the probe sequence on the promoter would anneal to a target polynucleotide of interest because of the complementarity (total or substantial, as the case may be) between the probe sequence and the target polynucleotide of interest, but would not anneal to the other polynucleotides not of interest for lack of complementarity. The annealing conditions, the transcription conditions, the length and/or the composition can be adjusted to increase or decrease the selectivity of the mobile promoter structure.

Because a mobile promoter structure by itself cannot support transcription (except synthesis of the bridge sequence), and because an annealed complex (which will support transcription from the mobile promoter) can be formed only if a target polynucleotide of interest is present in a test sample, the mobile promoter dependent transcription process can be used as a method for detecting the presence and quantity of a target polynucleotide in the test sample.

In a preferred embodiment, the test sample contains no "endogenous" promoters which are related to the mobile promoter structure to be used in the detection test. An "endogenous" promoter is related to the mobile promoter structure if the RNA polymerase to be used to transcribe an annealed complex would also transcribe a transcription unit comprising the endogenous promoter. Moreover, any other endogenous transcription capability would be disabled, for example, by prior deproteinization. Thus, in a preferred embodiment, there is mobile promoter structure directed transcription, and only such transcription. An example of a preferred embodiment of this type is where the mobile promoter structure is a highly specific prokaryotic promoter, and the test sample contains polynucleotides of eukaryotic origin. Examples of highly specific prokaryotic promoters are bacteriophage promoters from T3, T7 and SP6.

It is not critical that a test mixture supports only mobile promoter structure directed transcription. For example, a test mixture may support (i) mobile promoter structure directed transcription; (ii) hybrid transcription wherein the RNA polymerase used for mobile promoter structure dependent transcription may use certain endogenous sequences in the test mixture as promoters; and (iii) non-specifically initiated RNA synthesis (Sharmeen and Taylor, Nucl. Acids Res. 15: 6705-6711 (1987); Schenborn and Mierendorf, Nucl. Acids Res. 13: 6223-6236 (1985). In this situation, the transcript carrying the information encoded in the target polynucleotide of interest may nevertheless be detected by virtue of certain other specifiable characteristics, for example, a specific size.

In another embodiment of the invention, genetic information of interest embodied in a characteristic of the target polynucleotide of interest is elicited through mobile promoter structure directed transcription, followed by further characterization of the transcription products.

For example, a wild type gene may have a restriction site at a specific location, which is missing in a variant gene. Then, if the genomes of a wild type organism and a variant organism are digested with the appropriate restriction enzyme, the wild type gene fragment of interest will have a different size than the corresponding variant gene fragment. The mobile promoter structure directed transcripts obtained from the two organisms would also be different in size.

Another example is where no well-characterized differences between a wild type gene and a variant gene are known, or where, if known, no convenient method exists to cause the difference to be manifest. Here, the wild type gene of interest and the corresponding variant gene may be subjected to mobile promoter structure directed transcription as described herein above. The wild type and variant transcripts are then sequenced by any suitable techniques, and the gene sequences compared.

Many sequencing techniques are now available. The sequencing technique of Sanger et al. and its variants have been described, and are well known in the art of molecular genetics. See, for example, Sanger et al., Proc. Natl. Acad. Sci. USA 74:5463 (1977). Alternatively, the mobile promoter structure directed transcripts may be sequenced directly. See, for example, Gish and Eckstein, Science 240: 1520-1522 (1988). These two papers on sequencing are incorporated herein by reference.

In another embodiment, the transcript produced under the direction of a mobile promoter structure is reverse transcribed by primer extension synthesis. Primer extension synthesis has been described in Feinberg and Vogelstein, Analytical Biochem. 137: 266-267 (1984). This process in combination with mobile promoter dependent synthesis would produce amplification of a polynucleotide sequence complementary to a target polynucleotide sequence. If necessary, the transcript may be preferentially digested to leave only the reverse transcript as the product of the combination process. For example, the transcript may be RNA, and the reverse transcript may be DNA. The transcript can be digested by a RNAse.

In another embodiment of the invention, a first mobile promoter structure directs the synthesis of a transcript of a first target polynucleotide sequence. The transcript is reverse transcribed by primer extension synthesis. (The primer for reverse transcription can be chosen to hybridize to any desired portion of the transcript.) The transcript-reverse transcript duplex is melted to its single stranded components. The reverse transcript has the same sequence as the first target polynucleotide sequence, and is now available as a second target polynucleotide sequence. A second mobile promoter structure suitable for the second target polynucleotide sequence (i.e., having a probe sequence which would form a stable hybrid to permit mobile promoter structure directed transcription) is provided to direct transcription of a second transcript. This process is iterated. The first, second, third, .... transcripts all comprise the sequence of the first target polynucleotide. Thus, this iterative process permits exponential (relative to the number of cycles of mobile promoter structure directed transcription) amplification of a target polynucleotide sequence. This iterative process is illustrated schematically in Figure 9.

The first, second, third, .... mobile promoter structures can, but need not be distinct. Generally, two mobile promoter structures will suffice. In one embodiment, the first mobile promoter structure has no bridge sequence, and the whole series of mobile promoter structures are the same.

In another embodiment of the invention, the transcript of a mobile promoter structure directed transcription reaction serves as a target polynucleotide of interest in a "second round" transcription directed by a second mobile promoter structure. Here again the process can be iterated. An iterative process is illustrated schematically in Figure 10.

In a preferred embodiment, a ligation reaction precedes each new round of transcription because ligated annealed complexes are more efficient than unligated complexes.

In another embodiment, the first mobile promoter structure has no bridge sequence.

Following are examples which illustrate procedures, including the best mode, for practicing the invention. These examples should not be construed as limiting. All percentages are by weight and all solvent mixture proportions are by volume unless otherwise noted.

Example 1 - Formation Of A Mobile Promoter Structure

A first DNA sequence:
5′-AATTAACCCTCACTAAAGGGAACCTGCGCAAGGAACGCCCGTCGTGGC-3′ was duplexed with a second DNA sequence:
3′-TTAATTGGGAGTGATTTCCCTTG-5′ to form a mobile promoter:
5′-AATTAACCCTCACTAAAGGGAACCTGCGCAAGGAACGCCCGTCGTGGC-3′
3′-TTAATTGGGAGTGATTTCCCTTG -5′
which is useful for directing transcription by the RNA polymerase of bacteriophage T3.

The double-stranded sequence:
5′-AATTAACCCTCACTAAA-3′
3′-TTAATTGGGAGTGATTT-5′
is a natural T3 RNA polymerase promoter (Basu et al. 1984 JBC 259, 1993-1998).

The duplex 5′-GGGAAC-3′
3′-CCCTTG-5′
is formed by the plus bridge sequences 5′-GGGAAC-3′, and the minus bridge sequence 3′-CCCTTG-5′.

The DNA sequence consisting of the last 25 nucleotides on the 3′ end of the first DNA sequence, namely, 5′-CTGCGCAAGGAACGCCCGTCGTGGC-3′, forms a probe sequence which is complementary to a sequence of the neo gene of the bacterial transposon Tn5.

Example 2 - Formation Of An Annealed Complex Which is Transcriptionally Competent

The mobile promoter structure of Example 1 was annealed to a target DNA sequence of interest - a 270 base sequence of the neo gene of Tn5. The 270 base sequence was obtained by melting a 270 bp PvuII-BgIII restriction fragment of a plasmid containing the neo gene (Pharmacia), pZL250 (pAC 362) (Loewy et al. [1987] Genes and Development 1:626-635). The annealed complex is shown illustratively in Figure 1. This complex is transcriptionally competent.

Example 3 - Formation of A Ligated Annealed Complex

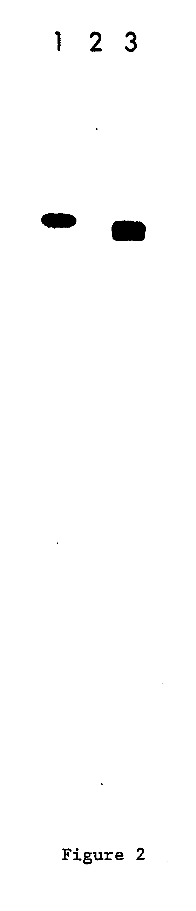

The annealed complex of Example 2 was incubated overnight with T4 DNA ligase at 37 C to generate a ligated annealed complex in which the nick between the 5$'$ terminal nucleotide of the minus strand of the mobile promoter structure, and the 3$'$ terminal nucleotide of the target neo gene sequence is repaired, and replaced by a 3$'$-5$'$ phosphodiester bond. The 5$'$ terminal nucleotide of the minus strand of the mobile promoter structure was labeled with T4 polynucleotide kinase and $^{32}$P ATP. Figure 2 demonstrates the formation of the annealed complex. Lane 1 corresponds to a 270 base size standard. Lane 2 shows the labeled oligonucleotide alone, and lane 3 shows the labeled oligonucleotide ligated to the 270 base target sequence for the neo gene. The formation of a ligated annealed complex was indicated by the shift in electromobility of radioactive DNA in a denaturing gel, from one corresponding to low molecular weight to one corresponding to about 300 bases.

Example 4 - Mobile Promoter Structure Directed Transcription

The ligated annealed complex of Example 3 was incubated with T3 polymerase in the presence of ribonucleoside triphosphates. Reaction conditions for the in vitro transcriptions were performed essentially as described (Melton et al. [1984] Nucleic Acids Res. 12:4035). Following the transcription reaction, 2 $\mu$l of tRNA (10 mg/ml) and 90 $\mu$l of 4 M glacial acetic acid were added to each tube and the tubes were placed on ice for 10 minutes to precipitate the RNA. The pellets were collected and a second acetic acid precipitation was performed with the addition of 200 $\mu$l of 2 M glacial acetic acid, followed by a 70% ethanol wash. The pellets were resuspended in loading dye (90% formamide; 1X TBE; 0.02% bromophenol blue; 0.02% xylene cyanol) and analyzed on a 6% polyacrylamide 7 M urea gel.

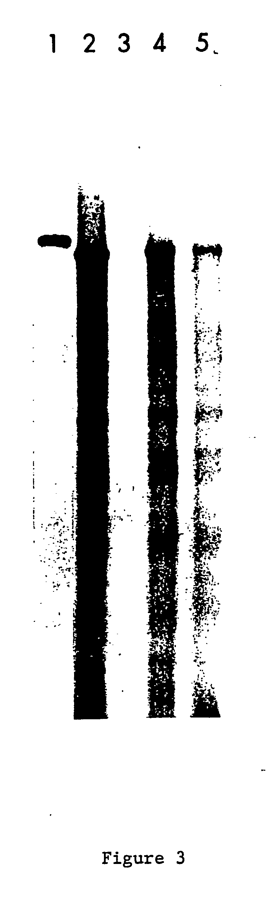

Figure 3 illustrates the products of a T3 mobile promoter structure transcription reaction. Lane 1 contained a 270 base size standard. The full size transcripts from mobile promoter structure directed transcription are expected to run at nearly the same position as the material in lane 1. Indeed, lane 2 contained the products of the transcription reaction and showed a prominent band at the expected position, although products of smaller sizes were also evident. Lanes 3 and 4 contained the transcription reaction products treated with RNAse and DNAse, respectively, prior to electrophoresis. The results clearly indicate that the transcription reaction products were RNA. Lane 5 shows that the transcription reaction products can bind to an anion exchange resin (Quiagen). RNA binds specifically to this resin, whereas DNA and proteins are eluted off the column. Collectively, these results demonstrate that the product obtained from the transcription reaction was indeed RNA.

Other polymerases, for example, T7 RNA polymerase and SP6 RNA polymerase can be used in the above reaction to give essentially the same results.

Example 5 - Efficiency Of Mobile Promoter Structure Directed Transcription Complexes

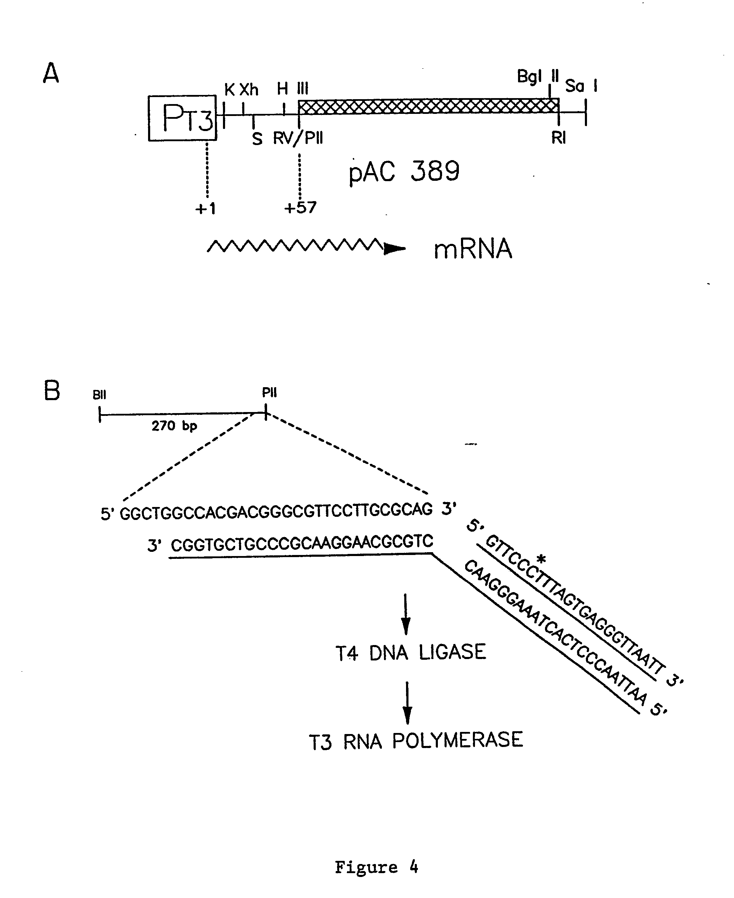

In this example, the efficiency of a mobile promoter structure directed transcription complex was compared with that of a similar complex in a "cloned" system. pAC389 is a plasmid containing a cloned T3 promoter upstream of the same sequences recognized by the mobile promoter structure. pAC389 was obtained by isolating the 270 base pair EcoRI/PvuII fragment from the neo gene (Fig. 1) (which can be obtained from Pharmacia) and ligating this fragment into a Bluescript vector (Stratagene) that was restricted with EcoRI and EcoRV. The RNA strand generated from pAC389 is the same strand obtained from the mobile promoter structure transcription of pAC362. The relevant portion of pAC389 is shown in Figure 4A.

The ligated annealed complex of Example 3 is also shown for comparison (4B). The site of initiation of transcription is represented by the asterisk above the (-) strand oligonucleotide. The nucleotide sequences of the two transcription "units" differ only in a small polylinker sequence (57 nucleotides long) which is present in pAC389 (Figure 4A).

Table 1 identifies the presence of transcription products when a given amount of linearized pAC389 or mobile promoter structure-neo gene complex were used as the transcription templates. The results reflect the appearance of a transcript and demonstrate that the two types of transcription complexes have comparable efficiencies.

Table 1

| Sensitivity[a] of the T3 Mobile Promoter Structure | | | | | |
|---|---|---|---|---|---|
| Transcription Unit | Template Concentration | | | | |
| | 27 ng | 5.4 ng | 0.5 ng | 50pg | 1pg |
| T3 Mobile Promoter Structure | + | + | + | - | - |
| pAC389 | + | + | + | - | - |

[a]Sensitivity was determined by detecting the presence ( + ) or absence (-) of a transcript.

Example 6 - Amplification Of The Neo Gene Sequence

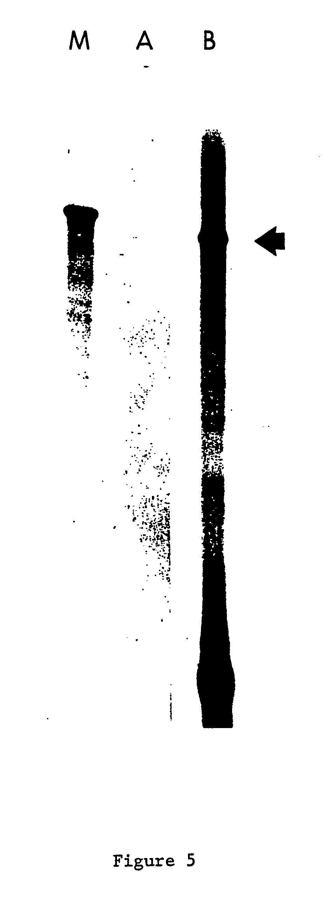

In this example, an equivalent amount of neo gene target sequence was used to form a ligated transcription competent complex as was used as a template for primer extension synthesis. One copy of a primer extension per copy of template can be produced; however, one template in a transcription complex can produce many transcripts. The ratio of radioactive precursor incorporation, after allowing for the difference in specific activities, is a measure of the amplification of a gene by mobile promoter structure-directed transcription. Figure 5 demonstrates the magnitude of standard amplification obtained with the mobile promoter structure. A 270 base size standard is shown in the land designated M. The primer extension product is shown in lane A and the product of a T3 mobile promoter structure transcription is shown in lane B. Approximately fifty-fold more product is generated as a result of a transcription reaction than is generated by primer extension.

Example 7 - Selectivity Of A Mobile Promoter Structure

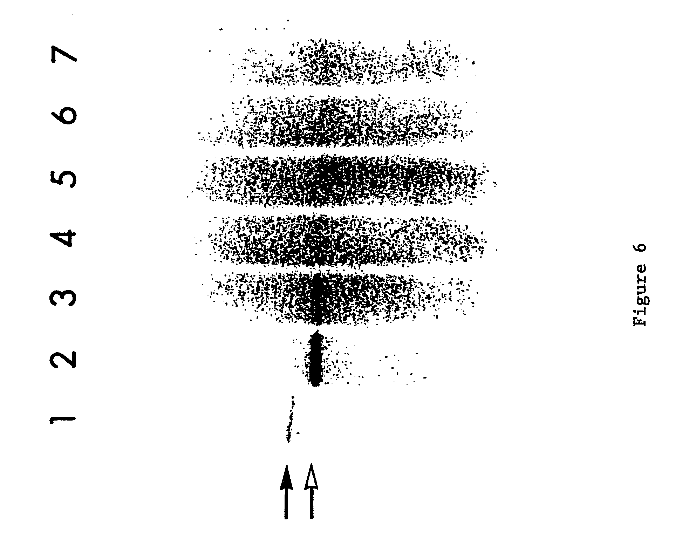

Amounts of neo gene sequences varying from 1 pg to 500ng in the form of mobile promoter structure transcription complexes were mixed with 1 ug of total human DNA. Human genomic DNA was isolated essentially as has been described (Kunkel, L.M. et al. [1982] Nucl. Acids. Res. 10:1557-1578). The target sequence DNA as well as the human genomic DNA was digested with BglII and PvuII. The target sequence DNA (neo sequences), human genomic DNA, and the appropriate oligonucleotides which comprise the T3 promoter, were incubated at 100°C for 10 minutes and annealed slowly to 37°C. This complex was incubated overnight at 37°C with T4 DNA ligase to repair the nick (Figure 1). These mixtures were incubated in transcription reactions, as described (Example 4). Figure 6 shows that the T3 specific transcripts can be detected when the transcription reaction was carried out in a 1-million-fold excess non-specific background.

Example 8 - Detection of the Presence of a Specific Genomic Sequence

Using a Mobile Promoter Structure of Formula (A) (Detection of Sickle Cell Genotype)

The mobile promoter structure:
consisting of (a) the bacteriophage T3 promoter:

5′-AATTAACCCTCACTAAA-3′
3′-TTAATTGGGAGTGATTT-5′

(b) the bridge sequence:

5′-GGGAAC-3′
3′-CCCTTG-5′

and (c) the probe sequence

5′-CCAATCTACTCCCAGGAGCAGGG-3′

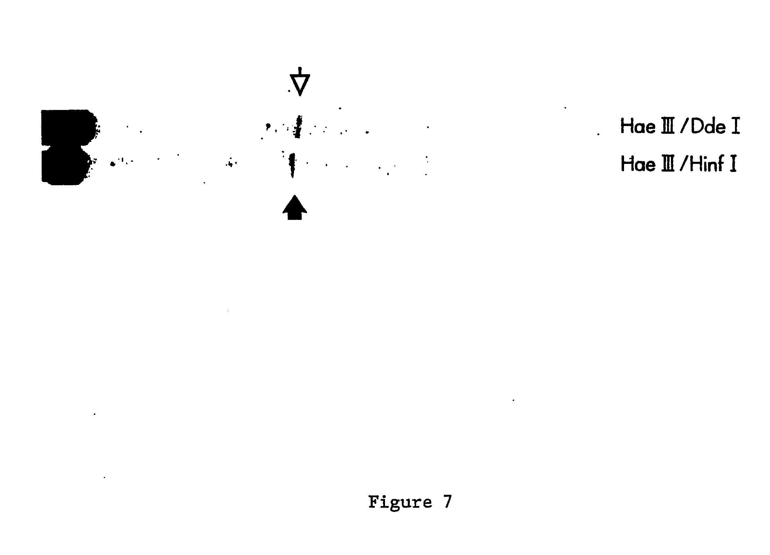

was annealed to a restriction digest of genomic DNA from a wild type individual. The genomic DNAs had been digested with HaeIII and HinfI or HaeIII and DdeI. The probe sequence is adjacent to the HaeIII site which is about 150 bases 5′ to the polymorphic DdeI site (Poncz et al., JBC 258: 11599-11609, 1983). Figure 7 illustrates the ligated annealed complexes of a wild type individual where the DNA was restricted with HaeIII/HinfI and HaeIII/DdeI respectively. The ligated complexes were electrophoresed on a denaturing polyacrylamide gel.

Example 9 - Mobile Promoter Structure Directed Transcription of a RNA Template

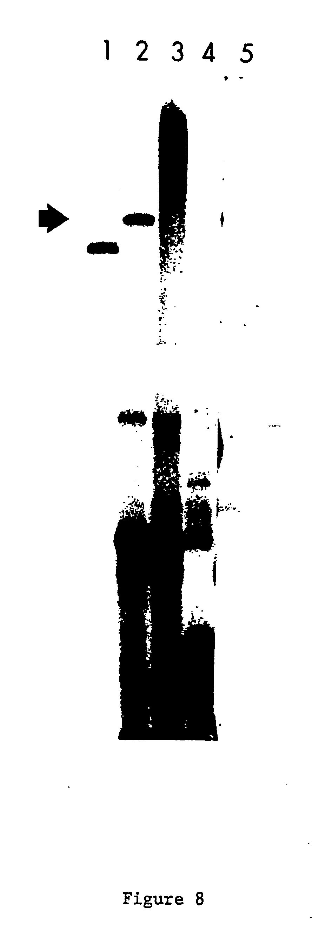

The mobile promoter structure of Example 1 was annealed to a RNA sequence that is complementary to the 270 base sequence of the neo gene of Tn5. The RNA template was obtained by transcribing a plasmid (pAC399) that encoded the neo gene under the control of the bacteriophage SP6 promoter. pAC399 was constructed by inserting the BglII/SalI fragment which encodes the neo gene into pSP65 (obtainable from Promega) which was restricted with BamHI and SalI. Transcription of pAC399 with SP6 RNA polymerase generated a mRNA which was subsequently isolated on a Quiagen column. The oligonucleotides which comprise the T3 promoter were annealed to the RNA and the complex was ligated with T4 RNA ligase at room temperature overnight. The ligated annealed complex was incubated with T3 RNA polymerase in the presence of ribonucleoside triphosphates. The reaction conditions were as previously described (Melton et al. [1984] Nucl. Acids Res. 12:4035). Transcription products were precipitated with glacial acetic acid as described in Example 4 and fractionated on a 6% polyacrylamide 7M urea gel. The results are shown in Figure 8. Lane 1 corresponds to a 270 base size standard. Lane 2 corresponds to the transcription product of a RNA/DNA transcription complex transcribed with T3 RNA polymerase. Lane 3 corresponds to the identical transcription complex transcribed with T7 RNA polymerase. Lane 4 corresponds to the transcription complex transcribed with T3 RNA polymerase, in the presence of a limiting nucleotide concentration (no riboCTP was added). Lane 5 corresponds to the transcription complex transcribed in the absence of any polymerase. These results suggest that in the presence of T3 RNA polymerase, RNA can serve as a suitable template for transcription.

Example 10 - Kit for Amplifying and/or Detecting Nucleic Acid Sequences

For convenience and standardization, reagents for amplifying and/or detecting nucleic acid sequences can be assembled in a kit. Such a kit can comprise, in a packaged form, a multicontainer unit having

(a) a container containing a first polynucleotide sequence comprising a plus promoter sequence, a plus bridge sequence, and a probe sequence, wherein (i) the plus promoter sequence is linked and 5′ to the plus bridge sequence, and the plus bridge sequence is linked and 5′ to the probe sequence, (ii) the plus bridge sequence is equal to or greater than zero nucleotides long, and (iii) the 3′ terminus of the first polynucleotide sequence is the 3′ terminus of the probe sequence;

(b) a container containing a second polynucleotide sequence comprising a minus promoter sequence, wherein (i) the minus promoter sequence is linked and 3′ to the minus bridge sequence, (ii) the minus promoter sequence is complementary to the plus promoter sequence, (iii) the minus bridge sequence is complementary to the plus bridge sequence, and (iv) the 5′ terminus of the second polynucleotide sequence is the 5′ terminus of the minus bridge sequence; and

(c) a container containing means for detecting hybrids of said probe sequence.

The above kit components can be readily adapted into an apparatus for laboratory use.

Also, as will be readily apparent to a person skilled in this art, the subject invention processes can be

EP 0 369 775 A2

automated to provide desired nucleic acid sequences in a continuous form.


**Claims**

1. A mobile promoter structure comprising:

(a) a first polynucleotide sequence comprising, sequentially-linked, a plus promoter sequence, an optional plus bridge sequence, and a probe sequence at the 3′-terminus of the first polynucleotide sequence; and

(b) a second polynucleotide sequence comprising a minus promoter sequence complementary to the plus promoter sequence, and an optional minus bridge sequence complementary to the optional plus bridge sequence, wherein the 5′-terminus of the second polynucleotide sequence is the 5′-terminus of the minus promoter sequence or, if present, the minus bridge sequence.

2. A mobile promoter structure according to claim 1, wherein the plus bridge sequence is present and is at least one nucleotide long.

3. A mobile promoter structure according to claim 2, wherein the plus bridge sequence is at least 6 nucleotides long.

4. A mobile promoter structure according to claim 1, which is
5′-AATTAACCCTCACTAAAGGGAACCTGCGCAAGGAACGCCCGTCGTGGC-3′
3′-TTAATTGGGAGTGATTTCCCTTG -5′.

5. An annealed complex comprising a mobile promoter structure according to any of claims 1 to 4, and a target polynucleotide of which at least the 3′-terminal portion is single-stranded.

6. An annealed complex according to claim 5, wherein the 5′-terminus of the second polynucleotide sequence of the mobile promoter and the 3′-terminus of the target polynucleotide are ligated.

7. A method of transcribing a target polynucleotide sequence, comprising:

(a) incubating a mobile promoter structure according to any of claims 1 to 4 with the target polynucleotide, under conditions such that they form an annealed complex; and

(b) incubating the annealed complex under conditions which give transcription under the direction of the mobile promoter structure.

8. A method according to claim 7, wherein the target polynucleotide is part of a double-stranded polynucleotide, and which comprises the preliminary additional step of separating the two strands by denaturation.

9. A method according to claim 7 or claim 8, wherein step (b) is conducted in the presence of an RNA polymerase selected from T3 RNA polymerase, T7 RNA polymerase and SP6 RNA polymerase.

10. A method according to any of claims 7 to 9, which additionally comprises, after step (a) but prior to step (b), ligating the 5′-terminus of the second polynucleotide sequence and the 3′-terminus of the target polynucleotide.

11. A method of transcribing polynucleotide sequences, comprising performing n (n being an integer of at least two) cycles of mobile promoter structure-directed transcription, wherein each cycle comprises a method according to claim 10, and wherein, in the $n^{th}$ cycle, the probe sequence anneals with the 3′ region of a target polynucleotide which is complementary to the target polynucleotide of the $(n-1)^{th}$ cycle.

12. A method of transcribing and amplifying a target polynucleotide sequence, comprising performing alternating cycles of mobile promoter structure-directed transcription according to the method of any of claims 7 to 10, and reverse transcription by primer extension synthesis.

13. A method according to claim 12, which comprises at least 2 cycles of mobile promoter structure-directed transcription.

14. A method according to any of claims 11 to 13, wherein all of the plus promoters are identical.

15. A method according to any of claims 7 to 14, which additionally comprises determining the size of, and/or sequencing, a transcription product, thereby characterising the target polynucleotide.

16. A method for transcribing an RNA template which comprises (1) annealing an RNA sequence with a mobile promoter, e.g. according to any of claims 1 to 4; (2) ligating the resulting complex, e.g. with T4 RNA polymerase; and (3) incubating the ligated annealed complex with an RNA polymerase, e.g. T3 RNA polymerase, in the presence of ribonucleoside triphosphates.

17. A method according to claim 16, wherein the RNA sequence is complementary to the 270 base sequence of the neo gene of Tn5.

12

Figure 1

1  2  3

Figure 2

1  2  3  4  5

Figure 3

**A**

K Xh    H III                                                Bgl II  Sa I

PT3

S   RV/PII                                                        RI

pAC 389

+1        +57

∿∿∿∿∿∿∿∿∿▶    mRNA

**B**

BII                    PII

270 bp

5' GGCTGGCCACGACGGGCGTTCCTTGCGCAG 3'

3' CGGTGCTGCCCGCAAGGAACGCGTC

5' GTTCCCTTTAGTGAGGGTTAATT 3'
                                              *
CAAGGGAAATCACTCCCAATTAA 5'

T4 DNA LIGASE

T3 RNA POLYMERASE

Figure 4

Figure 5

Figure 6

Hae Ⅲ /Dde Ⅰ

Hae Ⅲ /Hinf Ⅰ

Figure 7

1 2 3 4 5

Figure 8

Figure 9

EP 0 369 775 A2

Figure 10